# EUROPEAN PATENT APPLICATION

(11) **EP 3 566 715 A1**
(43) Date of publication of application: **13.11.2019**
(21) Application number: 19167809.3
(22) Date of filing: 23.10.2013
(51) Int. Cl.: A61K 39/145, A61K 9/06, A61K 9/72, A61K 47/32, A61P 31/16

(54) **NASAL INFLUENZA VACCINE COMPOSITION**

(30) Priority: 28.12.2012 JP 2012287900
(62) Divisional of application: 13867863.6
(71) Applicant: Japan as Represented by Director-General of National Institute of Infectious Diseases, Tokyo 162-8640 (JP); Toko Yakuhin Kogyo Kabushiki Kaisha, Kita-ku Osaka-shi Osaka 530-0022 (JP)
(72) Inventor: Hasegawa, Hideki, Tokyo 162-8640 (JP); Suzuki, Tadaki, Tokyo 162-8640 (JP); Ainai, Akira, Tokyo 162-8640 (JP); Kamishita, Taizou, Osaka 530-0022 (JP); Miyazaki, Takashi, Osaka 530-0022 (JP)
(74) Representative: Hoffmann Eitle

(57) **Abstract**

The invention relates to an influenza vaccine composition for spray-administration to nasal mucosa, which comprises an inactivated whole influenza virion and a gel base material comprising carboxy vinyl polymer, which is characterized by not comprising an adjuvant.

## Description

### TECHNICAL FIELD

The present invention relates to an influenza vaccine composition for spray-administration to nasal mucosa.

### BACKGROUND ART

Influenza is an acute respiratory tract infection caused by the influenza virus, in particular, influenza becomes epidemic in winter year after year. In addition, influenza sometimes results in a pandemic, and many people become severe to result in death. For influenza, it is known that the vaccination with influenza vaccine can bring in some preventive effects, thus people are broadly vaccinated before the epidemic season.

The influenza vaccine approved in Japan is only an inactivated protein-component of an influenza viral antigen to be subcutaneously vaccinated, and currently, a split vaccine thereof is used as the seasonal influenza vaccine. Such vaccine to be subcutaneously vaccinated is highly effective for preventing the severity in influenza infection such as pneumonia, but it has low antibody-induced activity in upper respiratory mucous membrane that is an infected area of influenza virus, which is not enough as infective protection-activity. And, such injection administration has problems, for example, a pain and side-effects such as inflammation caused by topical vaccination.

For the above problem of influenza vaccination, a wide variety of the trials have been done until now, in which a vaccine for nasal administration has received attention as a new vaccination. However, it has been reported that it is impossible to induce a high immune response to the influenza virus even though the split vaccine which has been broadly used in current clinical practice is nasally administered to experimental animals or human beings directly.

Under such circumstances, the world's first split influenza vaccine for nasal administration which comprises *Escherichia coli* heat labile toxin as an adjuvant was approved in Switzerland [Berna Biotech, Switzerland; Commercial name: Nasalflu], and the sale thereof started in October, 2000, but the clinical use thereof was withdrawn in February, 2004 due to the toxicity of the adjuvant. And, Patent Reference 1 also discloses an influenza vaccine for nasal administration which comprises an adjuvant, which indicates that the immune induction can be enhanced by using the adjuvant. However, the toxicity of adjuvants is an anxious matter for practical use.

For the nasal administration, it is also necessary to consider the complicated structure of nasal cavity, and it is desirable to make influenza vaccine broadly spread, attached and retained for a long time in nasal cavity. For example, the base (material) disclosed in Patent Reference 2 may be used for spray-administration.

As mentioned above, it has been desired to develop influenza vaccine for nasal administration as a next-generation influenza vaccine and put it to practical use, which takes the place of a conventional influenza vaccine for subcutaneous or intramuscular administration. However, there are various problems for the practical use, for example, how the toxicity of an adjuvant used to enhance the immune induction should be reduced.

### PRIOR ART

### [Patent Reference]

[Patent Reference 1] WO 2010/114169
[Patent Reference 2] WO 2007/123193

### SUMMARY OF INVENTION

One of the purposes of the present invention is to provide an influenza vaccine composition for spray-administration to nasal mucosa which is prepared by using an inactivated whole influenza virion as an antigen that has been already approved, but not using an adjuvant, which exhibits a high efficacy and low side effects in spite of a low antigen level; a process thereof; and a method for preventing influenza.

The present inventors have extensively studied on the above problem and have found that a combination of (i) a gel base (material) for spray-administration to nasal mucosa comprising carboxy vinyl polymer which is treated by adding an outside shearing force to add spray-performance and (ii) an inactivated whole influenza virion, can enhance the immune induction in human beings without an adjuvant. Based upon the new findings, the present invention has been accomplished. The present invention may provide the following embodiments.
[1] An influenza vaccine composition for spray-administration to nasal mucosa comprising
   (i) an inactivated whole influenza virion, and
   (ii) a gel base material comprising carboxy vinyl polymer which is treated by adding an outside shearing force to add spray-performance,
   which does not comprise an adjuvant.
[2] The influenza vaccine composition for spray-administration to nasal mucosa of [1]
   wherein the amount of (i) the inactivated whole influenza virion is 1 - 500 µg HA/mL per type of vaccine virus strain.
[3] The influenza vaccine composition for spray-administration to nasal mucosa of [1] or [2] which comprises 0.1 w/v % to 1.0 w/v % carboxy vinyl polymer.
[4] The influenza vaccine composition for spray-administration to nasal mucosa of any one of [1] to [3] wherein the spray-performance is to control (1) the particle-size-distribution of the sprayed composition, (2) the uniformity of spray density, and/or (3) the spray angle.
[5] The influenza vaccine composition for spray-administration to nasal mucosa of any one of [1] to [4] which is prepared by
   treating a gel base material comprising 0.5 w/v % to 2.0 w/v % carboxy vinyl polymer by adding an outside shearing force to control (1) the particle-size-distribution of the sprayed composition, (2) the uniformity of spray density, and/or (3) the spray angle, as spray-performance, to give a gel base material for spray-administration, and then
   mixing the resulting gel base material with a virus stock solution comprising an inactivated whole influenza virion homogeneously in a short time without stress.
[6] The influenza vaccine composition for spray-administration to nasal mucosa of any one of [1] to [5] which is prepared with a gel base material for spray-administration comprising carboxy vinyl polymer that is treated by adding an outside shearing force to add spray-performance which is to control that
   (1) as for the particle-size-distribution of the sprayed composition, the mean particle size is in a range of 30 µm to 80 µm, and the particle distribution between 10 µm and 100 µm is 80 % or more,
   (2) the spray density is uniform to form a homogeneous full-corn shape, and
   (3) the spray angle is adjusted in a range of 30° to 70°.
[7] The influenza vaccine composition for spray-administration to nasal mucosa of any one of [1] to [5] which is prepared with a gel base material for spray-administration comprising carboxy vinyl polymer that is treated by adding an outside shearing force to add spray-performance which is to control that
   (1) as for the particle-size-distribution of the sprayed composition, the mean particle size is in a range of 40 µm to 70 µm, and the particle distribution between 10 µm and 100 µm is 90 % or more,
   (2) the spray density is uniform to form a homogeneous full-corn shape, and
   (3) the spray angle is adjusted in a range of 40° to 60°.
[8] The influenza vaccine composition for spray-administration to nasal mucosa of any one of [1] to [7] which comprises a gel base material for spray-administration comprising carboxy vinyl polymer which is treated by adding an outside shearing force to control (1) the particle-size-distribution of the sprayed composition, (2) the uniformity of spray density, and (3) the spray angle, in order to enable a sprayable device without a pumping function to make a spray-administration.
[9] A method for preventing influenza comprising administering the influenza vaccine composition for spray-administration to nasal mucosa according to any one of [1] to [8] to a subject in need using a device which can spray a viscous formulation from naris to intranasal mucosa.
[10] Use of the influenza vaccine composition for spray-administration to nasal mucosa according to any one of [1] to [8] for preventing influenza.

### [Effect of the Invention]

The present invention have made it possible to provide an influenza vaccine composition for spray-administration to nasal mucosa comprising an inactivated whole influenza virion as an active ingredient, but not comprising an adjuvant, which induces a high immune response in spite of a small antigen level, and low side effects because the composition does not comprise an adjuvant. The influenza vaccine composition is expected to be suitably used for the epidemic of influenza.

The influenza vaccine composition for spray-administration to nasal mucosa of the present invention can be broadly spread, attached and retained for a long time in nasal mucosa because the composition comprises a gel base material for spray-administration to nasal mucosa comprising carboxy vinyl polymer which is treated by adding an outside shearing force to add spray-performance, thus the influenza vaccine composition of the present invention can induce a high immune response in spite of a small antigen level.

According to the process for preparing an influenza vaccine composition for spray-administration to nasal mucosa of the present invention, an influenza vaccine composition for spray-administration to nasal mucosa can be provided, which well keeps the antigenicity of the inactivated whole virion because the virion is treated in a short time without stress, and induces a high immune response and low side effects.

Although the present invention comprises no adjuvant as an immunopotentiating agent, the present invention can provide an equal or more potent immune-induction for upper respiratory mucous membrane and whole body, compared with a composition comprising influenza virus vaccine and an adjuvant.

### DESCRIPTION OF EMBODIMENTS

The present invention provides an influenza vaccine composition for spray-administration to nasal mucosa comprising a gel base material for spray-administration to nasal mucosa comprising carboxy vinyl polymer which is treated by adding an outside shearing force to add spray-performance, and an inactivated whole influenza virion, which is characterized by not comprising an adjuvant.

The "gel base material comprising carboxy vinyl polymer which is treated by adding an outside shearing force to add spray-performance" used herein means, for example, a "gel base material comprising a skin/mucosa-adhesive agent" disclosed in WO 2007/123193, which is a base material comprising carboxy vinyl polymer and optionally comprising gellan gum, whose viscosity is adjusted by adding an outside shearing force. The base material is characterized in that the viscosity thereof can be adjusted to various ones by adding an outside shearing force, and the spray spreading-angle from a spray container and the spray density can be controlled to meet the purpose. For the inoculation to many people in developing countries and the pandemic, a upper-pressure-relief airless-type spray container disclosed in WO 2007/123193 and WO 2007/123207 as a multiple-dose spray container can be used for the purpose because the spray container can be set to be sprayed at any angle or any angle-range and can be used up without leftover in the container. When such gel base material for spray-administration in the present invention is used, the spreadability and adhesivability of an inactivated whole influenza virion at nasal mucosa can be enhanced widely over a long time frame, thereby the immunogenicity of the vaccine can be enhanced.

Carboxy vinyl polymer which is a material of the base material for spray-administration to nasal mucosa in the present invention is a hydrophilic polymer prepared by polymerizing acrylic acid as a main ingredient, which can use without any limitation pharmaceutical additives that are generally used to prepare an aqueous gel agent.

The content of the gel base material comprising carboxy vinyl polymer which is treated by adding an outside shearing force to add spray-performance is 0.1 - 1.0 w/v %, preferably 0.3 - 0.7 w/v % as the content of carboxy vinyl polymer.

The vaccine of the present invention is characterized by comprising an inactivated whole influenza virion as an antigen. The inactivated whole influenza virion used herein means a virion which is prepared by cultivating influenza virus to give a virus suspension thereof and purifying the virus suspension while keeping its virus morphology. Thus, the influenza vaccine of the present invention means a vaccine except split vaccine (including subvirion) and subunit vaccine (including purified HA or NA), and it is also referred to as whole virus vaccine.

The above-mentioned inactivated whole influenza virion is preferably such virion that is purified from a virus suspension in the absence of surfactants and ethers. The virus stock solution used herein means a virus solution comprising an inactivated whole influenza virion, which is purified or concentrated to be mixed with a gel base material for spray-administration to nasal mucosa. With regard to the vaccine of the present invention, the concentration of an inactivated whole influenza virion is preferably 1 - 500 µg HA/mL (in HA equivalent), more preferably 20 - 250 µg HA/mL (in HA equivalent) per type of vaccine virus strain. The above-mentioned concentration can be determined by measuring the concentration of HA protein.

The influenza virus used herein includes all types of currently-known influenza virus and all subtypes thereof, as well as all types and all subtypes of influenza virus isolated or identified in future. In addition, from the viewpoint of the necessity to also effectively prevent an infection that has not become epidemic in human beings until now, but might become epidemic in human beings in future, a combination of an influenza A virus subtype selected from the group consisting of subtypes H1 - H16 excluding subtype H1 and H3 (*i.e*., H2, and H4 - H 16) and an influenza A virus subtype selected from the group consisting of subtypes N1 - N9 is preferable. These subtypes are also referred to as a new type influenza virus. As the above-mentioned subtypes, a combination of a subtype selected from the group consisting of subtypes H5, H7, and H9 and a subtype selected from the group consisting of subtypes N1 - N9 is more preferable. The influenza virus may be derived from a type of strain, two or more types of strains belonging to the same subtype, or two or more types of strains belonging to different subtypes.

The influenza virus used herein includes a strain isolated from infected animals or humans, and a recombinant virus genetically-established at cultured cells. As the method for cultivating influenza virus, the virus may be seeded in the allantoic cavity of eggs of hen and cultivated, or may be infected in cultured cells and cultivated.

An adjuvant is a generic term of substances having the modulating-activity of the immune response such as enhancement and suppression, and is used as an immunopotentiating agent to be added to a vaccine to enhance the immunogenicity of an antigen. Until now, a lot of adjuvants have been studied. The use of an adjuvant enhances the immune effect of a vaccine, but it has disadvantages of side effects such as inflammation. Some adjuvants can be chosen as a candidate to be used in a vaccine for nasal administration, but there has not been any approved vaccine for nasal administration comprising an adjuvant because there has been no adjuvant having a pervasive safety.

The present inventors have found that it is possible to prepare a vaccine having a high efficacy and low side effects in spite of non-adjuvant and a lower antigen level when the gel base material for spray-administration to nasal mucosa which has the above-mentioned useful spray-performance such as high adhesive property to nasal mucosa is used with the above-mentioned whole-virus vaccine. In addition, the present inventors have also found that using a device which can spray even a gel base material having high viscosity, an influenza vaccine composition for spray-administration to nasal mucosa wherein the mean particle size of the sprayed composition is in a suitable range of 30 µm to 80 µm (preferably a range of 40 µm to 70 µm), the particle-size-distribution between 10 µm and 100 µm is 80 % or more (preferably, 90 % or more), the spray angle from the device is set at a range of 30° to 70° (preferably, a range of 40° to 60°) so that the composition can be administered to the desired site in nasal cavity, and the spray density is uniform to form a homogeneous full-corn shape, can be sprayed to nasal mucosa. Further the present inventors have also found its process and a method for preventing influenza using the composition. Based upon the new findings, the present invention has been accomplished.

The vaccine of the present invention can comprise an additional pharmaceutically-acceptable carrier(s) besides an inactivated whole influenza virion and a gel base material for spray-administration to nasal mucosa. The carrier used herein can be a carrier which is generally used in the preparation of a vaccine or a formulation for administration in nasal cavity, which includes, for example, saline, buffered saline, dextrose, water, glycerin, isotonic aqueous buffer solution, and a combination thereof. And, the vaccine of the present invention may optionally include a preservative (e.g. thimerosal), an isotonic agent, a pH regulator, a surfactant, and an inactivating agent (e.g. formalin).

The vaccine of the present invention is used for spray-administration into the nasal cavity.

The vaccine of the present invention can prevent influenza or relieve the symptom thereof.

For the administration of the vaccine, a multiple-dose spray container or a sprayable device to both nares without a pumping function, in general, a disposable sprayable device to both nares without a pumping function can be used.

The dosage of the vaccine should be decided considering the age, sex and weight of a patient or other factors, and actually the vaccine can be administered once or more times in an amount of generally 1 µg HA - 150 µg HA, preferably 5 µg HA - 50 µg HA as an antigen per type of vaccine virus strain. Preferably, the vaccine is administered in plural. In this case, the interval of the administration is preferably 1 to 4 weeks.

### EXAMPLE

Hereinafter, the invention is illustrated based on examples, but are not limited thereto.

According to the methods shown below, a gel base material and three kinds of virus stock solutions were prepared, and the gel base material and each virus stock solution were mixed as shown below to prepare influenza vaccine compositions as examples.

### <Preparation of gel base material>

### Example of gel base material (1)

| Ingredients | Amount | Process of Preparation |
|---|---|---|
| Carboxy vinyl polymer | 11.0 mg | Each ingredient shown in the left column was mixed in the ratio corresponding to each weight shown there, and stirred to become homogeneous. Then, the mixture was given an outside shearing force by a high-speed rotation with an intermittently-jet-stream-generating-type high-speed spinning-type emulsifying device. The resulting base material whose viscosity was suitably adjusted with an outside shearing force was heated at 90°C for 20 minutes to give a gel base material for spray-administration. |
| L-arginine | 24.0 mg | |
| Concentrated glycerin | 20.0 mg | |
| Purified water | q.s. | |
| Total | 1.0 mL | |
| | | Aspect: a clear and colorless gel base material, almost odorless. |
| | | pH: 7.15 |
| | | Viscosity: 4,000 mPa·s |

### <Preparation of virus stock solution comprising inactivated whole influenza virion>

### Example of virus stock solution (1)

| Ingredients | Amount | Process of Preparation |
|---|---|---|
| Inactivated whole antigen of influenza virus A/Victoria/210/2009 (H3N2) | 180 µg HA | The strain for preparing the vaccine was seeded in the allantoic cavity of embryonated eggs and cultivated, and then the virus suspension was collected. In order to clarify the virus suspension, it was centrifuged or filtrated, and ultrafiltered to be concentrated. Then, in order to purify the virus, the filtrate was ultracentrifuged by, for example, sucrose density gradient centrifugation to give a purified virus solution. The purified virus solution was inactivated with formalin to give a purified inactivated virus solution. And then, the solution was ultrafiltered to give a virus stock solution. |
| Sodium hydrogen phosphate hydrate | 3.53 mg | |
| Sodium dihydrogen phosphate | 0.54 mg | |
| Sodium chloride | 8.50 mg | |
| Purified water | Total 1.0 mL | |

### Example of virus stock solution (2)

| Ingredients | Amount | Process of Preparation |
|---|---|---|
| Inactivated whole antigen of influenza virus A/Indonesia/5/05 (H5N1) | 180 µg HA | The strain for preparing the vaccine was seeded in the allantoic cavity of embryonated eggs and cultivated, and then the virus suspension was collected. In order to clarify the virus suspension, it was centrifuged or filtrated, and ultrafiltered to be concentrated. Then, in order to purify the virus, the filtrate was ultracentrifuged by, for example, sucrose density gradient centrifugation to give a purified virus solution. The purified virus solution was inactivated with formalin to give a purified inactivated virus solution. And then, the solution was ultrafiltered to give a virus stock solution. |
| Sodium hydrogen phosphate hydrate | 3.53 mg | |
| Sodium dihydrogen phosphate | 0.54 mg | |
| Sodium chloride | 8.50 mg | |
| Purified water | Total 1.0 mL | |

### Example of virus stock solution (3)

| Ingredients | Amount | Process of Preparation |
|---|---|---|
| Inactivated whole antigen of influenza virus A/California/7/2009 (H1N1)pdm09 | 60 µg HA | The strain for preparing the vaccine was seeded in the allantoic cavity of embryonated eggs and cultivated, and then the virus suspension is collected. In order to clarify the virus suspension, it was centrifuged or filtrated, and ultrafiltered to be concentrated. Then, in order to purify the virus, the filtrate was ultracentrifuged by, for example, sucrose density gradient centrifugation to give a purified virus solution. The purified virus solution was inactivated with β-propiolactone and formalin to give a purified inactivated virus solution. And then, the solution was ultrafiltered to give a virus stock solution. |
| Inactivated whole antigen of influenza virus A/Victoria/365/2011 (H3N2) | 60 µg HA | |
| Inactivated whole antigen of influenza virus B/Wisconsin/01/2010 | 60 µg HA | |
| Sodium hydrogen phosphate hydrate | 3.53 mg | |
| Sodium dihydrogen phosphate | 0.54 mg | |
| Sodium chloride | 8.50 mg | |
| Purified water | Total 1.0 mL | |

### <Mixture of gel base material and virus stock solution>

Example of gel base material (1) and each of Examples of virus stock solution (1) - (3) mentioned above were mixed in the ratio of 1:1 under stirring to give each homogeneous influenza vaccine composition, Examples 1, 2, and 3, respectively. The compositions of each Example and their physical properties/spray-performances are shown below. The mixing under stirring can be completed softly and in a short time without stressing the inactivated whole antigen of virus. The quantities of each ingredient in the resulting influenza vaccine compositions, the physical properties thereof, and the spray-performances thereof derived by spraying the compositions with a suitable device are also shown below.

### Example 1

| Ingredients | Amount | Physical property/spray-performance |
|---|---|---|
| Inactivated whole antigen of influenza virus A/Victoria/210/2009(H3N2) | 90 µg HA | pH: 7.25 |
| Carboxy vinyl polymer | 5.50 mg | Viscosity: 500 mPa·s |
| L-arginine | 12.00 mg | Spray-performance in spraying 250 µL of the solution with a device without a pumping function: |
| Concentrated glycerin | 10.00 mg | |
| Sodium hydrogen phosphate hydrate | 1.765 mg | |
| Sodium dihydrogen phosphate | 0.270 mg | • Mean particle size of sprayed formulation: 52.7 µm |
| Sodium chloride | 4.25 mg | • Ratio of particle size between 10 µm and 100 µm: 91.5 % |
| Purified water | q.s. | |
| Total | 1.0 mL | • Spray angle from the device: 53° |
| | | • Spray density: full-corn uniformly-circle |

### Example 2

| Ingredients | Amount | Physical property/spray-performance |
|---|---|---|
| Inactivated whole antigen of influenza virus A/Indonesia/5/05(H5N1) | 90 µg HA | pH: 7.10 |
| Carboxy vinyl polymer | 5.50 mg | Viscosity: 430 mPa·s |
| L-arginine | 12.00 mg | Osmotic pressure: 293 mOsm Spray-performance in spraying 250 µL of the solution with a device without a pumping function: |
| Concentrated glycerin | 10.00 mg | |
| Sodium hydrogen phosphate hydrate | 1.765 mg | |
| Sodium dihydrogen phosphate | 0.270 mg | • Mean particle size of sprayed formulation: 55.2 µm |
| Sodium chloride | 4.25 mg | |
| Purified water | q.s. | • Ratio of particle size between 10µm and 100 µm: 95.0 % |
| Total | 1.0 mL | • Spray angle from the device: 51° |
| | | • Spray density: full-corn uniformly-circle |

### Example 3

| Ingredients | Amount | Physical property/spray-performance |
|---|---|---|
| Inactivated whole antigen of influenza virus A/Calfornia/7/2009(H1N1)pdm0 9 | 30 µg HA | pH: 7.15 |
| | | Viscosity: 520 mPa·s |
| | | Osmotic pressure: 295 mOsm Spray-performance in spraying 250 µL of the solution with a device without a pumping function: |
| Inactivated whole antigen of influenza virus A/Victoria/365/2011 (H3N2) | 30 µg HA | |
| Inactivated whole antigen of influenza virus B/Wisconsin/01/2010 | 30 µg HA | • Mean particle size of sprayed formulation: 57.4 µm |
| Carboxy vinyl polymer | 5.50 mg | |
| L-arginine | 12.00 mg | • Ratio of particle size between 10 µm and 100µm: 95.0 % |
| Concentrated glycerin | 10.00 mg | • Spray angle from the device: 52° |
| Sodium hydrogen phosphate hydrate | 1.765 mg | |
| Sodium dihydrogen phosphate | 0.270 mg | • Spray density: full-corn uniformly-circle |
| Sodium chloride | 4.25mg | |
| Purified water | q.s. | |
| Total | 1.0 mL | |

As an influenza vaccine composition without a gel base material, Comparative examples 1 - 4 were prepared according to the compositions shown in the following tables by optionally using the inactivated whole antigen used in the above examples.

### Comparative example 1

| Ingredients | Amount |
|---|---|
| Inactivated split antigen of influenza virus A/Uruguay/716/2007(H3N2) | 90 µg HA |
| Sodium hydrogen phosphate hydrate | 3.53 mg |
| Sodium dihydrogen phosphate | 0.54 mg |
| Sodium chloride | 8.50 mg |
| Purified water | q.s. |
| Total | 1.0 mL |

### Comparative example 2

| Ingredients | Amount |
|---|---|
| Inactivated whole antigen of influenza virus A/Indonesia/5/05(H5N1) | 90 µg HA |
| Sodium hydrogen phosphate hydrate | 3.53 mg |
| Sodium dihydrogen phosphate | 0.54 mg |
| Sodium chloride | 8.50 mg |
| Purified water | q.s. |
| Total | 1.0 mL |

### Comparative example 3

| Ingredients | Amount |
|---|---|
| Inactivated whole antigen of influenza virus A/Calfornia/7/2009(H1N1)pdm09 | 30 µg HA |
| Inactivated whole antigen of influenza virus A/Victoria/365/2011 (H3N2) | 30 µg HA |
| Inactivated whole antigen of influenza virus B/Wisconsin/01/2010 | 30 µg HA |
| Sodium hydrogen phosphate hydrate | 3.53 mg |
| Sodium dihydrogen phosphate | 0.54 mg |
| Sodium chloride | 8.50 mg |
| Purified water | q.s. |
| Total | 1.0 mL |

### Comparative example 4

| Ingredients | Amount |
|---|---|
| Inactivated split antigen of influenza virus A/Calfornia/7/2009(H1N1)pdm09 | 30 µg HA |
| Inactivated split antigen of influenza virus A/Victoria/365/2011(H3N2) | 30 µg HA |
| Inactivated split antigen of influenza virus B/Wisconsin/01/2010 | 30 µg HA |
| Sodium hydrogen phosphate hydrate | 3.53 mg |
| Sodium dihydrogen phosphate | 0.54 mg |
| Sodium chloride | 8.50 mg |
| Purified water | q.s. |
| Total | 1.0 mL |

### Test for Evaluating Immune Response (1)

With each influenza vaccine composition prepared in Example 1 and Comparative example 1, two groups composed of 4 adult volunteers in each group were vaccinated by nasal spray-administration with an appropriate disposable device, in an amount of 0.25 mL for one nostril (equivalent of 45 µg HA for both nostrils), twice at an interval of 3 weeks.

The blood and the washings of nasal cavity were consecutively collected, and the neutralizing antibody titer thereof for vaccine strain was measured and analyzed. The results are shown in Table 1 for Example 1, and Table 2 for Comparative example 1.

**Table 1**

| No. | Sex | Neutralizing antibody titer in serum | | | Neutralizing antibody titer in washings of nasal cavity | | |
|---|---|---|---|---|---|---|---|
| | | Initial (pre) | 3 weeks later | 6 weeks later | Initial (pre) | 3 weeks later | 6 weeks later |
| 01 | M | 80 | ≥1280 | ≥1280 | 40 | 640 | 640 |
| 02 | M | 5 | 5 | 40 | <20 | <20 | 40 |
| 03 | F | 20 | 160 | 320 | <20 | 40 | 40 |
| 04 | F | 640 | ≥1280 | ≥1280 | 40 | 40 | 160 |

**Table 2**

| No. | Sex | Neutralizing antibody titer in serum | | | Neutralizing antibody titer in washings of nasal cavity | | |
|---|---|---|---|---|---|---|---|
| | | Initial (pre) | 3 weeks later | 6 weeks later | Initial (pre) | 3 weeks later | 6 weeks later |
| 01 | M | 40 | 160 | 160 | 20 | 80 | 160 |
| 02 | M | <10 | <10 | 10 | 20 | 20 | 80 |
| 03 | M | 20 | 20 | 20 | 40 | 80 | 320 |
| 04 | M | <10 | <10 | <10 | 20 | 20 | 80 |

Comparing the results of the vaccine of Example 1 (the virus stock solution + the gel base material for spray-administration) and the vaccine of Comparative example 1 (a composition comprising the inactivated split antigen of influenza virus without the gel base material), the neutralizing antibody titer in serum of 3/4 subjects vaccinated with the vaccine of Comparative example 1 did not increase, while the neutralizing antibody titer in serum of 4/4 subjects vaccinated with the vaccine of Example 1 increased, and that significantly increased. The neutralizing antibody titer in washings of nasal cavity increased in all cases about both the vaccines of Example 1 and Comparative example 1, but the vaccine of Example 1 showed greater increase.

### Test for Evaluating Immune Response (2)

With each influenza vaccine composition prepared in Example 2 and Comparative example 2, two groups composed of 25 adult volunteers for Example 2 and 24 adult volunteers for Comparative example 2 were vaccinated by nasal spray-administration with an appropriate disposable device, in an amount of 0.25 mL for one nostril (equivalent of 45 µg HA for both nostrils), twice at an interval of 3 weeks, and one more time about a half year later, totally three times.

The blood and the washings of nasal cavity were collected 3 weeks after the third vaccination, and the neutralizing antibody titer thereof to vaccine strain was measured and analyzed. The results are shown in Table 3.

**Table 3**

| | Variation of neutralizing antibody titer to A/Indonesia/5/05(H5N1) | | | | | | | |
|---|---|---|---|---|---|---|---|---|
| | Serum | | | | Washings of nasal cavity | | | |
| | Example 2 | | Comparative example 2 | | Example 2 | | Comparative example 2 | |
| | pre | post | pre | post | pre | post | pre | post |
| Geometric mean titer* (GMT) | 5.0 (<10) | 164.5 | 5.0 (<10) | 84.8 | 10.0 (<20) | 105.6 | 10.0 (<20) | 46.2 |
| GMT percentage of rise | | 32.9 | | 17.0 | | 10.5 | | 4.6 |

Comparing the results of the vaccine of Example 2 (the virus stock solution + the gel base material for spray-administration) and the vaccine of Comparative example 2 (only the virus stock solution), it was shown that the vaccine of Example 2 comprising the gel base material for spray-administration increased the immune response more greatly than that of Comparative example 2.

It is known that a human in a naive state who has never contacted influenza virus antigen (such as babies and children) induces less immune response. It is thought that the immune response in such susceptible individuals to influenza vaccine can be estimated by evaluating the immune response in healthy adults to the vaccine of highly pathogenic avian influenza virus (H5N1 stain) because almost all healthy adults have never contacted the avian influenza virus (*i.e*., in a naive state).

As shown in the above results, it has been found that even for susceptible individuals, the neutralizing antibody titer in serum and washings of nasal cavity can be induced in high level by nasally-vaccinating the vaccine of Example 2 (the virus stock solution + the gel base material for spray-administration) three times.

### Analytical Test of Immune Response (3)

With each influenza vaccine composition prepared in Example 3 and Comparative example 3, two groups composed of 26 adult volunteers for Example 3 and 25 adult volunteers for Comparative example 3 were vaccinated by nasal spray-administration with an appropriate disposable device, in an amount of 0.25 mL for one nostril (in total, 15 µg HA for both nostrils), twice at an interval of 3 weeks. And, with the influenza vaccine composition prepared in Comparative example 4 (currently-used vaccine), a group composed of 38 adult volunteers was subcutaneously vaccinated once in an amount of 0.5 mL (15 µg HA/strain/0.5 mL).

The blood and the washings of nasal cavity were collected 3 weeks after the final vaccination (2nd or 1st), and the neutralizing antibody titer thereof to vaccine strain was measured and analyzed. The results are shown in Table 4.

**Table 4**

| | Variation of neutralizing antibody titer to A/Victoria/365/2011(H3N2) | | | | | |
|---|---|---|---|---|---|---|
| | Neutralizing antibody titer in serum | | | | | |
| | Example 3 | | Comparative example 3 | | Comparative example 4 | |
| | nasal | | nasal | | subcutaneous | |
| | pre | post | pre | post | pre | post |
| Geometric mean titer* (GMT) | 55.1 | 182.8 | 62.3 | 146.0 | 121.7 | 297.5 |
| GMT percentage of rise | | 3.32 | | 2.34 | | 2.44 |

Comparing the results of the nasally-administered vaccine of Example 3 (the virus stock solution + the gel base material for spray-administration), the nasally-administered vaccine of Comparative example 3 (only the virus stock solution), and the subcutaneously-administered vaccine of Comparative example 4 (currently-used vaccine for subcutaneous-administration), it was shown that the nasally-administered vaccine of Example 3 comprising the gel base material for spray-administration increased the immune response more greatly than that of the nasally-administered vaccine of Comparative example 3 or that of the subcutaneously-administered vaccine of Comparative example 4.

The current invention is defined, *inter alia*, by the following items:
1. An influenza vaccine composition for spray-administration to nasal mucosa comprising
   (i) an inactivated whole influenza virion, and
   (ii) a gel base material comprising carboxy vinyl polymer which is treated by adding an outside shearing force to add spray-performance,
   which does not comprise an adjuvant.
2. The influenza vaccine composition for spray-administration to nasal mucosa of item 1
   wherein the amount of (i) the inactivated whole influenza virion is 1 - 500 µg HA/mL per type of vaccine virus strain.
3. The influenza vaccine composition for spray-administration to nasal mucosa of item 1 or 2 which comprises 0.1 w/v % to 1.0 w/v % carboxy vinyl polymer.
4. The influenza vaccine composition for spray-administration to nasal mucosa of any one of items 1 to 3 wherein the spray-performance is to control (1) the particle-size-distribution of the sprayed composition, (2) the uniformity of spray density, and/or (3) the spray angle.
5. The influenza vaccine composition for spray-administration to nasal mucosa of any one of items 1 to 4 which is prepared by
   treating a gel base material comprising 0.5 w/v % to 2.0 w/v % carboxy vinyl polymer by adding an outside shearing force to control (1) the particle-size-distribution of the sprayed composition, (2) the uniformity of spray density, and/or (3) the spray angle, as spray-performance, to give a gel base material for spray-administration, and then
   mixing the resulting gel base material with a virus stock solution comprising an inactivated whole influenza virion homogeneously in a short time without stress.
6. The influenza vaccine composition for spray-administration to nasal mucosa of any one of items 1 to 5 which is prepared with a gel base material for spray-administration comprising carboxy vinyl polymer that is treated by adding an outside shearing force to add spray-performance which is to control that
   (1) as for the particle-size-distribution of the sprayed composition, the mean particle size is in a range of 30 µm to 80 µm, and the particle distribution between 10 µm and 100 µm is 80 % or more,
   (2) the spray density is uniform to form a homogeneous full-corn shape, and
   (3) the spray angle is adjusted in a range of 30° to 70°.
7. The influenza vaccine composition for spray-administration to nasal mucosa of any one of items 1 to 5 which is prepared with a gel base material for spray-administration comprising carboxy vinyl polymer that is treated by adding an outside shearing force to add spray-performance which is to control that
   (1) as for the particle-size-distribution of the sprayed composition, the mean particle size is in a range of 40 µm to 70 µm, and the particle distribution between 10 µm and 100 µm is 90 % or more,
   (2) the spray density is uniform to form a homogeneous full-corn shape, and
   (3) the spray angle is adjusted in a range of 40° to 60°.
8. The influenza vaccine composition for spray-administration to nasal mucosa of any one of items 1 to 7 which comprises a gel base material for spray-administration comprising carboxy vinyl polymer which is treated by adding an outside shearing force to control (1) the particle-size-distribution of the sprayed composition, (2) the uniformity of spray density, and (3) the spray angle, in order to enable a sprayable device without a pumping function to make a spray-administration.
9. A method for preventing influenza comprising administering the influenza vaccine composition for spray-administration to nasal mucosa according to any one of items 1 to 8 to a subject in need using a device which can spray a viscous formulation from naris to intranasal mucosa.
10. Use of the influenza vaccine composition for spray-administration to nasal mucosa according to any one of items 1 to 8 for preventing influenza.

## Claims

1. An influenza vaccine composition for spray-administration to nasal mucosa comprising
(i) an inactivated whole influenza virion, and
(ii) a gel base material comprising carboxy vinyl polymer which is treated by adding an outside shearing force to add spray-performance,
which does not comprise an adjuvant.

2. The influenza vaccine composition for spray-administration to nasal mucosa of claim 1
wherein the amount of (i) the inactivated whole influenza virion is 1 - 500 µg HA/mL per type of vaccine virus strain.

3. The influenza vaccine composition for spray-administration to nasal mucosa of claim 1 or 2 which comprises 0.1 w/v % to 1.0 w/v % carboxy vinyl polymer.

4. The influenza vaccine composition for spray-administration to nasal mucosa of any one of claims 1 to 3 wherein the spray-performance is to control (1) the particle-size-distribution of the sprayed composition, (2) the uniformity of spray density, and/or (3) the spray angle.

5. The influenza vaccine composition for spray-administration to nasal mucosa of any one of claims 1 to 4 which is prepared by
treating a gel base material comprising 0.5 w/v % to 2.0 w/v % carboxy vinyl polymer by adding an outside shearing force to control (1) the particle-size-distribution of the sprayed composition, (2) the uniformity of spray density, and/or (3) the spray angle, as spray-performance, to give a gel base material for spray-administration, and then
mixing the resulting gel base material with a virus stock solution comprising an inactivated whole influenza virion homogeneously in a short time without stress.

6. The influenza vaccine composition for spray-administration to nasal mucosa of any one of claims 1 to 5 which is prepared with a gel base material for spray-administration comprising carboxy vinyl polymer that is treated by adding an outside shearing force to add spray-performance which is to control that
(1) as for the particle-size-distribution of the sprayed composition, the mean particle size is in a range of 30 µm to 80 µm, and the particle distribution between 10 µm and 100 µm is 80 % or more,
(2) the spray density is uniform to form a homogeneous full-corn shape, and
(3) the spray angle is adjusted in a range of 30° to 70°.

7. The influenza vaccine composition for spray-administration to nasal mucosa of any one of claims 1 to 5 which is prepared with a gel base material for spray-administration comprising carboxy vinyl polymer that is treated by adding an outside shearing force to add spray-performance which is to control that
(1) as for the particle-size-distribution of the sprayed composition, the mean particle size is in a range of 40 µm to 70 µm, and the particle distribution between 10 µm and 100 µm is 90 % or more,
(2) the spray density is uniform to form a homogeneous full-corn shape, and
(3) the spray angle is adjusted in a range of 40° to 60°.

8. The influenza vaccine composition for spray-administration to nasal mucosa of any one of claims 1 to 7 which comprises a gel base material for spray-administration comprising carboxy vinyl polymer which is treated by adding an outside shearing force to control (1) the particle-size-distribution of the sprayed composition, (2) the uniformity of spray density, and (3) the spray angle, in order to enable a sprayable device without a pumping function to make a spray-administration.

9. The influenza vaccine composition for spray-administration to nasal mucosa according to any one of claims 1 to 8 for use in a method for preventing influenza.

10. The influenza vaccine composition for spray-administration to nasal mucosa according to claim 9 for the use according to claim 9,
wherein the method for preventing influenza comprises administering the influenza vaccine composition to a subject in need using a device which can spray a viscous formulation from naris to intranasal mucosa.
